Europäisches Patentamt

European Patent Office

Office européen des brevets

(19)

(11) Publication number: **0 060 943**
B1

(12) **EUROPEAN PATENT SPECIFICATION**

(45) Date of publication of patent specification: **13.06.84**

(21) Application number: **81301191.3**

(22) Date of filing: **19.03.81**

(51) Int. Cl.³: **C 07 C 43/29,**
C 07 C 51/58, C 07 C 41/22

(54) Paraphenoxybenzotrichloride, its preparation and use.

(43) Date of publication of application:
29.09.82 Bulletin 82/39

(45) Publication of the grant of the patent:
13.06.84 Bulletin 84/24

(84) Designated Contracting States:
BE CH DE FR GB IT LI NL

(56) References cited:
EP - A - 0 009 205
DE - A - 2 619 489
DE - A - 2 707 232
US - A - 4 014 940

Chemical Abstracts vol. 44, no. 9, 1950
Columbus, Ohio, USA M. TOMITA et al.
"Synthesis of diphenyl ether-4-acetic acid"
column 4448 f-h

(73) Proprietor: OCCIDENTAL CHEMICAL
CORPORATION
P.O. Box 189
Niagara Falls New York 14302 (US)

(72) Inventor: Colson, James G.
52 Dan Troy
Williamsville New York 14221 (US)
Inventor: Cooke, Victor F. G.
340 Brookshire Road
Youngstown New York 14174 (US)
Inventor: Day, F. Howard
1602 North Fairlane Drive
Grifton North Carolina (US)

(74) Representative: Myerscough, Philip Boyd et al,
J.A.Kemp & Co. 14, South Square Gray's Inn
London, WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to p-phenoxybenzotrichloride and to the preparation thereof. The compound p-phenoxybenzotrichloride is useful as a chemical intermediate in the preparation of various pesticides and polymers.

Aromatic polyketone polymers having excellent melt processability are described in U.S. Patent 4,024,314. The preferred monomer for the preparation of such polyketone polymers is p-phenoxy-benzoylfluoride. This monomer is prepared by fluorination of p-phenoxybenzoylchloride. Various methods for the preparation of the p-phenoxybenzoylchloride are known in the literature. In general the known method prior processes which may be employed in the preparation of p-phenoxybenzoylchloride are multi-stepped processes which are costly and frequently complicated by the presence of undesirable impurities stemming from undesired side reactions or from the presence of reactants and/or catalysts employed.

It has now been found that p-phenoxybenzoylchloride may be prepared in excellent yield and high purity by a novel process involving the use of a heretofore unknown compound, p-phenoxybenzotrichloride.

Summary of the Invention

It has now been found that the novel compound p-phenoxybenzotrichloride may be prepared by a process which comprises the photo-chlorination reaction of p-phenoxytoluene with chorine preferably in the presence of a suitable non-reactive solvent.The compound p-phenoxybenzotrichloride, prepared in this manner, may be partially hydrolysed by reacion with a suitable acid or by reaction with water to produce p-phenoxybenzoylchloride. Thus is one aspect the present invention is directed to a novel compound, p-phenoxybenzotrichloride, and in a second aspect to a process for the preparation thereof. In still another aspect the present invention is directed to the preparation of p-phenoxybenzotrichloride as a step in the production of p-phenoxybenzoylchloride.

The preparation of p-phenoxybenzotrichloride is generally accomplished by irradiating mixtures of the reactants at the desired reaction temperatures until the desired degree of chlorination has been achieved. Reaction temperatures are generally in the range of from 0° Celsius to 180° Celcius and preferably in the range of from 20° Celsius up to the boiling point of the reaction mixture.

The reaction of chlorine with p-phenoxytoluene may be carried out neat or, preferably, in the presence of a non-reactive solvent. Suitable solvents include benzoic acid or a halogenated hydrocarbon solvent such as methylene chloride, chlorobenzene, polychlorinated benzenes and 1,1,2-tri-chloro-1,2,2-trifluoroethane, which tend to moderate or facilitate the reaction. A preferred solvent for this purpose is carbon tetrachloride. It has been found that when carbon tetrachloride is employed as a solvent in the present reaction the tendency toward undesired ring chlorination is lessened.

Typically the reaction is carried out by the addition of chlorine gas or sulfuryl chloride, with or without suitable catalyst to a suitably heated liquid reaction medium comprising p-phenoxytoluene in carbon tetrachloride solvent. It has been found that undesired ring chlorination may occur as full conversion of the p-phenoxybenzotrichloride intermediate is approached. Accordingly, maximum yields and product purity are achieved by carefully following the progress of the reaction and stopping the addition of chlorine before extensive ring chlorination occurs.

Actinic light is employed for promoting the chlorination reaction. Any of the well known light sources which will accelerate the chlorination of p-phenoxytoluene may be used, such as tungsten filament lamps, ultraviolet lamps, mercury vapor arc lamps, fluorescent lamps, ordinary light bulbs, and the like. The preferred irradiation is an ultraviolet light source.

The compound p-phenoxybenzotrichloride is a white crystalline solid at ordinary temperatures, having a melting point of 48—49.50° Celsius, and thus is readily purified by fractional crystallization and distillation.

The crystallization can be achieved by partial crystallization of the neat material or by crystallization from a suitable solvent. Distillation is preferably run under reduced pressure.

The p-phenoxybenzotrichloride may be partially hydrolyzed to form p-phenoxybenzoylchloride in high yields. The hydrolysation can be effected by a hydroxy donating system, catalyzed or non-catalyzed, which donates an oxygen replacement of two of the chlorines from the trichloromethyl group without forming an adduct with the desired acid chloride product. The following compounds have been found effective in hydrolyzing p-phenoxybenzotrichloride to p-phenoxybenzoylchloride: organic acids including substituted and unsubstituted aliphatic mono- and di- carboxylic acids, such as acetic, propionic, butyric, maleic, adipic, glutaric and malonic acids as well as substituted and unsubstituted aromatic mono- and di- carboxylic acids such as benzoic, p-phenoxybenzoic, terephthalic, isophthalic and phthalic acids; aqueous mineral acids, such as hydrochloric, hydrobromic, hydroiodic and hydrofluoric acids as well as dilute sulfuric and phosphoric acids; water; sulfonic acids such as methane sulfonic acids, benzene sulfonic acid, toluene sulfonic acid and the like; mixtures of Lewis acids, e.g. ferric chloride, aluminum chloride and zinc chloride, with water and/or the afore identified organic acids; and, mixtures thereof. Thus, in one embodiment, p-phenoxybenzotrichloride is contacted with an approximately equimolar amount of water. The amount of water may vary somewhat but is preferably within 10% of the equimolar amount. Lesser amounts will result in lower yields of the p-phenoxybenzoylchloride and greater amounts will result in a degree of hydrolysis to p-phenoxybenzoic acid. The reaction may be carried out at temperatures of room temperature (about 20° Celsius) and higher and will proceed most readily with external heat at a temperature in excess of 100° Celsius and preferably at a temperature in the range of 130° to 150° Celsius. Under autogenous pressure higher temperature may be employed, however, temperatures in excess of 180° Celsius provide little advantages and are not preferred. In a preferred mode it has been found that the incorporation of hydrochloric acid in the starting reaction medium, facilitates partial hydrolysis reaction with the unexpected advantage of less stringent temperature requirements. The amount of hydrochloric acid employed, based on hydrogen chloride content, may vary considerably. Conveniently, concentrated hydrochloric acid is employed in an amount sufficient to provide the desired molar amounts of water. Lesser strengths of hydrochloric acid may be employed if desired. Preferred temperature in this mode is in the range of 70° to 110° Celsius.

Alternatively the partial hydrolysis of p-phenoxybenzotrichloride may be effected by reaction with a mono- or di- carboxylic acid to produce a mixture of p-phenoxybenzoylchloride and an acid chloride derived from the carboxylic acid employed. A wide variety of carboxylic acids may be employed including both aliphatic and aromatic carboxylic acids. The reaction proceeds to high yields in an unexpectedly facile manner at ambient temperature and without the need for a catalyst. Higher temperatures may be employed but are not required. In a particularly advantageous mode, p-phenoxybenzoic acid is employed as the carboxylic acid with the result that no co-product is formed since both reactants are converted in the reaction to the desired product p-phenoxybenzoylchloride.

The proportions of carboxylic acid employed may vary, but are preferably within 10% of the equimolar amount based on the p-phenoxybenzotrichloride reactant.

In one mode of the invention the p-phenoxybenzotrichloride was completely hydrolyzed with water alone, or an aqueous base such as KOH, and a suitable co-solvent such as acetone or isopropyl alcohol to form the p-phenoxybenzoic acid, or salt thereof (which may then be neutralized to the acid), and then chlorinated, for example with thionyl chloride, to form the desired p-phenoxybenzoylchloride.

The following specific examples are provided to further illustrate this invention and the manner in which it may be carried out. In the examples, unless otherwise indicated, all parts and percentages are by weight and all temperatures are in degrees Celsius.

## Example 1
### Preparation of P-Phenoxybenzotrichloride

Ten parts of p-phenoxytoluene containing about 0.2% of N,N-di-methyl caproamide (a sequestering agent to remove any traces of iron) was charged to a reaction vessel and heated to about 120°C. The temperature was maintained at about 120°C. while the reaction mixture was irradiated with a mercury arc lamp and approximately 12 parts of chlorine were sparged into the mixture over a period of 4 hours. The progress of the reaction was monitored with periodic sampling and analysis and the chlorine flow was stopped when over-chlorination (i.e., ring chlorination) was observed. Product samples were treated with $CaO/Na_2S_2O_2$, filtered, and analyzed by gas chromatographic techniques. The yield of p-phenoxybenzotrichloride was 20% based on p-phenoxytoluene starting material.

## Example 2
### Preparation of P-Phenoxybenzotrichloride

A mixture of 5.0 parts of p-phenoxytoluene and 0.02 parts of N,N-dimethyl caproamide in 21.53 parts of carbon tetrachloride was charged to a reaction vessel and heated to about 95°C. The reaction mixture was irradiated with a mercury arc lamp and the temperature maintained at about 95°C. while about 54 parts of chlorine were sparged into the mixture over a period of about 3.5 hours. The progress of the reaction was monitored by periodic sampling and analysis and the chlorine flow was stopped when over-chlorination was observed. Product samples were treated with $CaO/Na_2S_2O_2$, filtered and analyzed by gas chromatographic techniques. The yield of p-phenoxybenzotrichloride, was greater than 70%, based on p-phenoxytoluene starting material.

Samples of the p-phenoxybenzotrichloride prepared by the methods of Examples 1 and 2 were purified by fractional crystallization from heptane and acetonitrile. The purified samples were characterized by a melting point of 48—49.50°C. and mass spectral data confirmed a molecular structure of $C_{13}H_9Cl_3O$.

## Example 3
### Preparation of P-Phenoxybenzoylchloride

A mixture of 5.75 parts of p-phenoxybenzotrichloride and 0.36 parts of water was charged to a reaction vessel and gradually heated. Reaction was observed to occur as the temperature of the mixture approached 120°C. with a rapid increase as the temperature approached 140°C. The reaction mixture was maintained at about 140°C. for about one hour, then vacuum stripped and the product analyzed by gas chromatographic techniques. Analysis (in area percent) indicated 83% p-phenoxybenzoylchloride and 9% of the p-phenoxybenzotrichloride starting material.

## Example 4

Preparation of P-Phenoxybenzoylchloride

A mixture of 5.75 parts of p-phenoxybenzotrichloride and 0.58 parts of concentrated (37%) hydrochloric acid (containing 0.36 parts of water) was charged to a reaction vessel and heated slowly. As the temperature approached 78°C., reaction was observed with evolution of HCl. Continued heating at temperatures of about 80°—85°C. was maintained for about two hours. Analysis of the product by gas chromatographic techniques indicated 36.0% of p-phenoxybenzoylchloride and 24.5% of p-phenoxybenzotrichloride starting material.

## Example 5

Preparation of P-Phenoxybenzoylchloride

P-phenoxybenzotrichloride (288 parts) was added to 182 parts of p-phenoxybenzoic acid, with mixing. The mixture was allowed to stand at ambient temperature for about 16 hours, then heated, over a period of about 4 hours, to a temperature of about 140°C., at which point HCl evolution was detected. The mixture was maintained at a temperature of between 112° and 140°C. for an additional 21 hours, then cooled and analyzed by gas chromatography techniques. Analysis indicated a 73% yield of p-phenoxybenzoylchloride based on the starting materials.

## Example 6

Preparation of P-Phenoxybenzoylchloride

A mixture of 5.75 parts of p-phenoxybenzotrichloride and 2.44 parts of benzoic acid was charged to a reaction vessel and heated to about 140°C. over a period of about one hour, at which point the evolution of HCl from the reaction mixture was observed. The mixture was maintained at 120° to 140°. for an additional hour, then cooled and analyzed by gas chromatographic techniques. Analysis indicated 19.9% p-phenoxybenzoylchloride, and 8.3% benzoylchloride.

## Example 7

Preparation of P-Phenoxybenzoylchloride

Acetic acid (2.1 parts) was mixed with p-phenoxybenzotrichloride (10 parts) and the mixture was allowed to stand at ambient conditions for about 64 hours. Analysius of the mixture by $C^{13}$ NMR analysis, in relative mole %, indicated 68% p-phenoxybenzoylchloride and 19% p-phenoxybenzotrichloride.

## Claims

1. p-Phenoxybenzotrichloride

2. A process for the preparaion of p-phenoxybenzotrichloride which comprises reacting p-phenoxytoluene with chlorine in the presence of actinic light.

3. A process according to claim 2 wherein the reaction is carried out in the presence of a solvent.

4. A process according to claim 3 wherein the solvent is a halogenated hydrocarbon.

5. A process according to claim 4 wherein the halogenated hydrocarbon is carbon tetrachloride.

6. A process for the preparation of p-phenoxybenzoylchloride characterised by reacting p-phenoxybenzotrichloride with a hydrolyzing reactant selected from water, aqueous mineral acids, carboxylic acids, sulfonic acids, mixtures of Lewis acids with water or carboxylic acids, and mixtures of two or more or these reactants.

7. A process according to claim 6 wherein the hydrolyzing reactant is water which is used in a molar ratio of water: p-phenoxybenzotrichloride of 0.9:1 to 1.1:1.

8. A process according to claim 6 wherein the hydrolyzing reactant is an aqueous mineral acid selected from hydrochloric, hydrobromic, hydroiodic, hydrofluoric and dilute sulfuric and phosphoric acids.

9. A process according to claim 6 wherein the hydrolyzing reactant is aqueous hydrochloric acid containing sufficient water to provide a molar ratio of water: p-phenoxybenzotrichloride of 0.9:1 to 1.1:1.

10. A process according to claim 6 wherein the hydrolyzing reactant is a carboxylic acid which is used in a molar ratio of carboxylic acid: p-phenoxybenzotrichloride of 0.9:1 to 1.1:1.

11. A process according to claim 6 to 10 wherein said carboxylic acid is selected from substituted and unsubstituted acetic, propionic, butyric, maleic, adipic, glutaric, malonic, benzoic, p-phenoxybenzoic, terephthalic, isophthalic and phthalic acids.

12. A process for the preparation of p-phenoxybenzoylchloride which comprises reacting p-phenoxytoluene with chlorine in the presence of actinic light to produce a product containing p-phenoxybenzotrichloride and hydrolyzing said p-phenoxybenzotrichloride with a hydrolyzing reactant selected from water, aqueous mineral acid, carboxylic acid, mixtures of Lewis acids with water or carboxylic acids and mixtures of at least two such hydrolyzing reactants to produce p-phenoxybenzoylchloride.

13. A process according to claim 12 wherein the product containing p-phenoxybenzotrichloride is separated from the reaction solution before the p-phenoxybenzotrichloride is hydrolyzed.

14. A process according to claim 13 wherein the separation is by crystallization or distillation.

**Revendications**

1. Trichloroparaphénoxytoluène.

2. Procédé pour la préparation du trichloroparaphénoxytoluène, qui consiste à faire réagir du p-phénoxytoluène avec du chlore en présence d'une lumière actinique.

3. Procédé selon la revendication 2, dans lequel la réaction est effectuée en présence d'un solvant.

4. Procédé selon la revendication 3, dans lequel le solvant est un hydrocarbure halogéné.

5. Procédé selon la revendication 4, dans lequel l'hydrocarbure halogéné est du tétrachlorure de carbone.

6. Procédé pour la préparation du chlorure de p-phénoxybenzoyl, caractérisé en ce qu'on fait réagir du trichloroparaphénoxytoluène avec un réactif d'hydrolyse choisi parmi l'eau, des acides minéraux aqueux, des acides carboxyliques, des acides sulfoniques, des mélanges d'acides de Lewis avec de l'eau ou des acides carboxyliques, et des mélanges de deux ou plusieurs de ces réactifs.

7. Procédé selon la revendication 6, dans lequel le réactif d'hydrolyse est de l'eau, qui est utilisée selon un rapport molaire d'eau: trichloroparaphénoxytoluène compris entre 0,9:1 et 1,1:1.

8. Procédé selon la revendication 6, dans lequel le réactif d'hydrolyse est un acide minéral aqueux choisi parmi les acides chlorhydrique, bromhydrique, iodhydrique, fluorhydrique et des acides sulfurique et phosphorique dilués.

9. Procédé selon la revendication 6, dans lequel de réactif d'hydrolyse est de l'acide chlorhydrique aqueux contenant suffisamment d'eau pour procurer un rapport molaire d'eau: trichloroparaphénoxytoluène compris entre 0,9:1 et 1,1:1.

10. Procédé selon la revendication 6, dans lequel le réactif d'hydrolyse est une acide carboxylique qui est utilisé dans un rapport molaire d'acide carboxylique: trichloroparaphénoxytoluène compris entre 0,9:1 et 1,1:1.

11. Procédé selon la revendication 6 ou la revendication 10, dans lequel l'acide carboxylique est choisi parmi les acides acétique, propionique, butyrique, maléique, adipique, glutarique, malonique, benzoïque, p-phénoxybenzoïque, téréphtalique, isophtalique et phtalique, substitués et non substitués.

12. Procédé pour la préparation du chlorure de p-phénoxybenzoyl, qui consiste à faire réagir du p-phénoxytoluène avec du chlore en présence d'une lumière actinique pour produire un produit contenant du trichloroparaphénoxytoluène et en hydrolysant ce trichloroparaphénoxytoluène avec un réactif d'hydrolyse choisi parmi l'eau, un acide minéral aqueux, un acide carboxylique, des mélanges d'acides de Lewis avec de l'eau ou des acides carboxylique, et des mélanges d'au moins deux tels réactifs d'hydrolyse, pour produire le chlorure de p-phénoxybenzoyl.

13. Procédé selon la revendication 12, dans lequel le produit contenant le trichloroparaphénoxytoluène est séparé de la solution de réaction avant l'hydrolyse du trichloroparaphénoxytoluène.

14. Procédé selon la revendication 13, dans lequel la séparation s'effectue par cristallisation ou distillation.

**Patentansprüche**

1. p-Phenoxybenzotrichlorid.

2. Verfahren zur Herstellung von p-Phenoxybenzotrichlorid durch Umsetzung von p-Phenoxytoluol mit Chlor in Gegenwart von aktinischem Licht.

3. Verfahren nach Anspruch 2, dadurch gekennzeichnet, daß die Umsetzung in Gegenwart eines Lösungsmittels durchgeführt wird.

4. Verfahren nach Anspruch 3, dadurch gekennzeichnet, daß als Lösungsmittel ein halogenierten Kohlenwasserstoff verwendet wird.

5. Verfahren nach Anspruch 4, dadurch gekennzeichnet, daß der halogenierte Kohlenwasserstoff Tetrachlorkohlenstoff ist.

6. Verfahren zur Herstellung von p-Phenoxybenzoylchlorid, dadurch gekennzeichnet, daß p-Phenoxybenzotrichlorid mit einem hydrolisierenden Reaktanten aus der Gruppe: Wasser, wasserhaltigen Mineralsäuren, Karboxylsäuren, Sulfonsäuren, Mischungen von Lewissäuren mit Wasser oder Karboxylsäuren, Mischungen von zwei oder mehreren dieser Reaktanten umgesetzt wird.

7. Verfahren nach Anspruch 6, dadurch gekennzeichnet, daß als hydrolisierender Reaktant Wasser in einem Molverhältnis Wasser: p-Phenoxybenzotrichlorid von 0.9:1 bis 1.1:1 verwendet wird.

8. Verfahren nach Anspruch 6, dadurch gekennzeichnet, daß der hydrolisierende Reaktant aus der nachfolgenden Gruppe wasserhaltiger Mineralsäuren ausgewählt wird: Chlorwasserstoff-, Bromwasserstoff-, Iodowasserstoff-, Fluorwasserstoff-, verdünnte Schwefel- und Phosphorsäuren.

9. Verfahren nach Anspruch 6, dadurch gekennzeichnet, daß die als hydrolisierender Reaktant verwendete Chlorwasserstoffsäure so viel Wasser enthält, daß sich ein Molverhältnis Wasser: p-Phenoxybenzotrichlorid von 0.9:1 bis 1.1:1 einstellt.

10. Verfahren nach Anspruch 6, dadurch gekennzeichnet, daß die als hydrolisierender Reaktant verwendete Karboxylsäure in einem Molverhältnis Karboxylsäure: p-Phenoxybenzotrichlorid von 0.9:1 bis 1.1:1 verwendet wird.

11. Verfahren nach Anspruch 6 oder 10, dadurch gekennzeichnet, daß als Karboxylsäure substituierte oder unsubstituierte Essig-, Propion-, Butter-, Malein-, Adipin-, Glutar-, Malon-, Benzoe-, p-Phenoxybenzoe-, Terephthal-, Isophthal- oder Phthalsäure verwendet wird.

12. Verfahren zur Herstellung von p-Phenoxybenzoylchlorid dadurch gekennzeichnet, daß p-Phenoxytoluol mit Chlor in Gegenwart von aktinischem Licht zu einem p-Phenoxybenzotrichlorid enthaltenden Produkt umgesetzt und dieses mit einem hydrolisierenden Reaktanten aus der Gruppe: Wasser, wasserhaltige Mineralsäure, Karboxylsäure, Mischungen von Lewissäuren mit Wasser oder Karboxylsäuren oder Mischungen von mindestens zwei dieser hydrolisierenden Reaktanten zu p-Phenoxybenzoylchlorid hydrolisiert wird.

13. Verfahren nach Anspruch 12, dadurch gekennzeichnet, daß das p-Phenoxybenzotrichlorid enthaltende Produkt vor der Hydrolyse des p-Phenoxybenzotrichlorids von der Reaktionslösung abgetrennt wird.

14. Verfahren nach Anspruch 13, dadurch gekennzeichnet, daß die Abtrennung durch Kristallisation oder Destillation erfolgt.